# FASCICULE DE BREVET EUROPEEN

(11) **EP 2 991 760 B1**
(45) Date de publication et mention de la délivrance du brevet: **06.04.2022**
(21) Numéro de dépôt: 14719802.2
(22) Date de dépôt: 29.04.2014
(51) Int. Cl.: B01J 20/18, B01J 20/28, B01J 20/30, B01J 20/32, C07C 7/13

(54) **ADSORBANTS ZÉOLITHIQUES COMPRENANT DE LA ZEOLITHE EMT, LEUR PROCÉDÉ DE PRÉPARATION ET LEURS UTILISATIONS**
ZEOLITHADSORPTIONSMITTEL MIT EMT-ZEOLITH, VERFAHREN ZUR HERSTELLUNG DAVON UND VERWENDUNGEN DAVON
ZEOLITE ADSORBENTS COMPRISING EMT ZEOLITE, METHOD FOR PREPARING SAME AND USES THEREOF

(30) Priorité: 30.04.2013 FR 1353971
(43) Date de publication de la demande: 09.03.2016
(73) Titulaire: IFP Energies nouvelles, 92500 Rueil-Malmaison (FR); ARKEMA FRANCE, 92700 Colombes (FR)
(72) Inventeur: BATS, Nicolas, F-69360 Saint Symphorien D'Ozon (FR); LAROCHE, Catherine, 69390 Vernaison (FR); LEFLAIVE, Philibert, F-69780 Mions (FR); BOUVIER, Ludivine, F-64300 Orthez (FR)
(74) Mandataire: Bandpay & Greuter
(86) Numéro de dépôt international: PCT/EP2014/058732
(87) Numéro de publication internationale: WO 2014/177567

(56) Documents cités:
- WO-A1-2008/009845
- US-A1- 2009 326 309
- ENG-POH NG ET AL: "Nucleation and Crystal Growth Features of EMT-Type Zeolite Synthesized from an Organic-Template-Free System", CHEMISTRY OF MATERIALS, vol. 24, no. 24, 21 décembre 2012 (2012-12-21), pages 4758-4765, XP055097228, ISSN: 0897-4756, DOI: 10.1021/cm3035455

## Description

### DOMAINE TECHNIQUE

L'invention concerne des adsorbants à base de cristaux agglomérés de zéolithe EMT comprenant du baryum ou du potassium ou du baryum et du potassium, leur procédé de préparation et leurs utilisations.

Ces adsorbants peuvent être utilisés plus particulièrement pour la production en phase liquide ou phase gaz de para-xylène très pur à partir d'une charge d'hydrocarbures aromatiques contenant des isomères à 8 atomes de carbone.

### TECHNIQUE ANTERIEURE

Il est connu dans l'art antérieur que des adsorbants comprenant des aluminosilicates cristallins peuvent être utilisés pour séparer certains hydrocarbures à partir de mélanges les contenant. Dans le domaine de la séparation d'hydrocarbures aromatiques et en particulier la séparation d'isomères en C8 aromatiques, il est généralement reconnu que l'utilisation de cations particuliers dans les sites cationiques d'aluminosilicates cristallins zéolithiques améliore la sélectivité de la zéolithe pour un des isomères en C8-aromatique. Cette adsorption différenciée au sein de la zéolithe permet la séparation des différents isomères en C8-aromatique, ce qui est utilisé industriellement pour la production de para-xylène très pur à partir d'une charge d'hydrocarbures aromatiques contenant des isomères à 8 atomes de carbone.

Ainsi, l'utilisation d'adsorbants zéolithiques constitués de zéolithes X ou Y comprenant, outre des cations sodium, des ions baryum, potassium ou strontium, seuls ou en mélanges, pour adsorber sélectivement en phase liquide le para-xylène dans un mélange d'hydrocarbures aromatiques, est bien connue de l'art antérieur.

Les brevets US 3 558 730, US 3 558 732, US 3 626 020, US 3 663 638 et US 3 960 774 montrent que des adsorbants zéolithiques comprenant des aluminosilicates de structure faujasite (FAU) à base de sodium et de baryum ou à base de sodium, de baryum et de potassium, sont efficaces pour la séparation du para-xylène présent dans des coupes aromatiques en C8 (coupes comprenant des hydrocarbures aromatiques à 8 atomes de carbone). Les adsorbants ci-dessus sont de préférence utilisés comme agents d'adsorption dans les procédés en phase liquide, notamment de type contre-courant simulé, similaires à ceux décrits dans le brevet US 2 985 589 et qui s'appliquent, entre autres, aux coupes aromatiques en C8.

Cependant, de manière générale, les propriétés d'adsorption des zéolithes pour les hydrocarbures aromatiques à 8 atomes de carbone (xylènes et éthylbenzène) varient de manière très fine en fonction de la taille et de la forme des pores ainsi que de la position des cations à l'intérieur de la structure qui influent à la fois sur le champ électrostatique présent à l'intérieur de la zéolithe et sur la forme du volume accessible dans les pores. D'autres paramètres, tels que la polarisabilité des cations et des molécules ou la flexibilité de la structure peuvent également avoir une influence. Il est donc extrêmement difficile de prévoir théoriquement et avec précision les caractéristiques d'adsorption d'une zéolithe vis-à-vis des hydrocarbures aromatiques à 8 atomes de carbone.

C'est ainsi par exemple que, pour améliorer la sélectivité d'adsorption de zéolithes ayant la structure faujasite pour les isomères aromatiques en C8, de nombreuses études ont fait mention de l'influence du rapport Si/AI de la zéolithe, de la nature des cations d'échange, ainsi que de leur teneur en eau. Mais il est très difficile de prédire le degré d'amélioration parce que ces facteurs exercent des actions combinées sur les caractéristiques d'adsorption des zéolithes.

Les facteurs importants qui influencent les performances d'un procédé de séparation par adsorption englobent notamment la sélectivité d'adsorption, la capacité d'adsorption et la cinétique de transfert de matière qui définit les vitesses d'adsorption et de désorption des différents composés. L'adsorbant doit donc présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (*ibid.,* page 243), que, dans le cas d'un adsorbant aggloméré, le transfert de matière global dépend de l'addition de la résistance diffusionnelle intra-cristalline et de la résistance diffusionnelle entre les cristaux. La résistance diffusionnelle intra-cristalline est proportionnelle au carré des rayons des cristaux et inversement proportionnelle à la diffusivité des molécules intracristalline.

La résistance diffusionnelle entre les cristaux (également appelée résistance macroporeuse) est quant à elle proportionnelle au carré des rayons des agglomérés et inversement proportionnelle à la diffusivité des molécules dans les macropores. Pour une structure zéolithique donnée, une taille d'aggloméré donnée et une température de fonctionnement donnée, les diffusivités sont fixées, et le seul moyen d'améliorer le transfert de matière consiste à réduire le diamètre des cristaux. Un gain sur le transfert global sera ainsi obtenu en réduisant la taille des cristaux.

Pour estimer cette amélioration de la cinétique de transfert, on peut utiliser la théorie des plateaux décrite par Ruthven dans « Principles of Adsorption and Adsorption Processes », (ibid., pages 248-250). Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Par conséquent, l'homme du métier s'attend à ce que des adsorbants zéolithiques agglomérés présentant à la fois une bonne capacité d'adsorption des xylènes et une bonne sélectivité pour le para-xylène, possèdent de très bonnes propriétés de séparation des xylènes lorsqu'ils sont réalisés à partir de petits cristaux de zéolithe dans les procédés en phase liquide de séparation du para-xylène contenu dans les coupes aromatiques en C8, par exemple de type contre-courant simulé. L'homme du métier est cependant dans l'impossibilité de définir a *priori* ou théoriquement et avec précision les caractéristiques d'adsorption d'une zéolithe particulière, notamment vis-à-vis des hydrocarbures aromatiques à 8 atomes de carbone.

La synthèse des zéolithes conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, et autres techniques d'agglomération connues de l'homme du métier. Ces agglomérés ne présentent pas les inconvénients inhérents aux matières pulvérulentes.

Ces agglomérés, qu'ils soient sous forme de plaquettes, de billes, d'extrudés, et autres, sont en général constitués de cristaux de zéolithe(s), qui constituent l'élément actif (au sens de l'adsorption) et d'un liant destiné à assurer la cohésion des cristaux sous forme d'agglomérés et de leur conférer une résistance mécanique suffisante pour résister aux vibrations et aux mouvements auxquels ils sont soumis au cours des opérations de séparations des isomères des coupes aromatiques en C8.

Cependant, les propriétés d'adsorption de ces agglomérés sont évidemment réduites par rapport à la poudre de cristaux, en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent des argiles appartenant à la famille de la kaolinite, et de préférence préalablement calcinés à des températures généralement comprises entre 500°C et 700°C.

La demande de brevet FR 2 789 914 décrit par exemple un procédé de fabrication d'agglomérés de zéolithe X, de rapport Si/AI compris entre 1,15 et 1,5, contenant du baryum et éventuellement du potassium. Les agglomérés ainsi obtenus, après zéolithisation du liant, présentent, du point de vue de l'adsorption du para-xylène contenu dans les coupes aromatiques en C8, des propriétés améliorées par rapport à des adsorbants préparés à partir de la même quantité de zéolithe X et de liant, mais dont le liant n'est pas zéolithisé. Les brevets US 2009/0326309 et WO 2008/009845 décrivent aussi des agglomérés de zéolithe X, contenant du baryum et/ou du potassium pour l'adsorption du para-xylène contenu dans les coupes aromatiques en C8.

De manière indépendante, la synthèse de cristaux de zéolithe EMT est également bien connue de l'art antérieur. En effet, depuis les travaux originaux de Guth et al. (F. Dougnier, J. Patarin, J.-L. Guth, D. Anglerot, Zeolites, 12, 160 (1992)), de nombreux travaux ont été consacrés à la synthèse de zéolithes ayant cette structure cristalline. La synthèse de la zéolite EMT pure est notamment possible en utilisant un agent structurant ("structure directing agent" ou "template" en anglais) tel que l'éther 18-couronne-6 et en contrôlant bien les paramètres de synthèse (EP0915808 et JP2012/218998).

De nombreuses études ont été rapportées dans la littérature afin de réduire la consommation de l'éther couronne, par exemple par recyclage après la synthèse (F. Dougnier, J. L. Guth, Microporous Mater., 6, 79 (1996)), ou à l'aide d'approches alternatives (voir par exemple le brevet NO 964988) et les publications suivantes : R. Wendelbo, M. Stocker, H. Junggreen, H. B. Mostad, D. E. Akporiaye, Microporous Mesoporous Mater., 28, 361 (1999) ; M. Matsukata, K. Kizu, M. Ogura, E. Kikuchi, Cryst. Growth Des., 1, 509 (2001); J. Sun, M. Sun, C. Nie, Q. Li, J. Chem. Soc. Chem. Commun., 2459 (1999) et T. Chatelain, J. Patarin, M. Soulard, J. L. Guth, P. Shulz, Zeolites, 15, 90, (1995). Plus récemment des travaux menés par Mintova et al. (Chem. Mater., (2012), 24, 4758-4765 ; et Science, Jan. 2012, 335, 70-73) ont démontré la possibilité d'obtenir des cristaux de zéolithe EMT sans utilisation d'agent structurant organique. En outre, les cristaux obtenus présentent comme caractéristiques un rapport Si/AI compris entre 1,0 et 1,4 ainsi qu'une taille nanométrique (typiquement comprise entre 6 et 15 nm), ce qui n'était pas le cas des synthèses précédentes, l'éther 18-couronne-6 stimulant plutôt la cristallisation de cristaux d'EMT de taille micrométrique.

La présente invention a pour objectif de fournir des nouveaux adsorbants à base de zéolithe EMT échangée au baryum ou au potassium, ou au baryum et potassium combinant :
○une bonne sélectivité,
○un bon transfert de matière et
○une bonne capacité d'adsorption,
pour la séparation du paraxylène contenu dans les coupes aromatiques en C8 dans un procédé de type contre-courant simulé.

Il a en effet été constaté, que de manière surprenante, une bonne capacité d'adsorption, un bon transfert de matière et une bonne sélectivité pour le paraxylène pouvaient être obtenus avec des adsorbants à base de zéolithe EMT échangée au baryum ou au potassium ou au baryum et potassium. Il est par ailleurs précisé que les expressions « compris entre... et... » et « de... à... » utilisées dans la présente description doivent s'entendre comme incluant chacune des bornes mentionnées, sauf mention contraire.

### RESUME DE L'INVENTION

L'invention concerne un adsorbant zéolithique comprenant des cristaux de zéolithe EMT et comprenant du baryum et/ou du potassium, dans lequel la teneur totale en oxydes d'ions alcalins ou alcalino-terreux autres que l'oxyde de baryum BaO et l'oxyde de potassium K₂O est comprise entre 0 et 5%, de préférence entre 0% et 2%, et de manière très préférée entre 0% et 1% en poids, bornes incluses, par rapport à la masse totale de l'adsorbant.

Les cristaux de zéolithes EMT ont avantageusement un rapport atomique Si/AI compris entre 1,00 et 2,00, de préférence entre 1,0 et 1,50, de manière plus préférée entre 1,05 et 1,50 et de manière encore plus préférée entre 1,10 et 1,50, bornes incluses.

Le diamètre moyen en nombre des cristaux de zéolithes EMT est avantageusement compris entre 5 nm et 1500 nm, de préférence compris entre 5 nm et 1000 nm, de manière encore plus préférée entre 10 nm et 500 nm, bornes incluses.

L'adsorbant selon l'invention peut comprendre également des cristaux d'au moins une autre zéolithe, choisie parmi les zéolithes de structure FAU, notamment LSX, MSX, X, Y ou EMC-1, ou choisie parmi les zéolithes de structure LTA ou MFI.

Dans un mode de réalisation, ladite ou lesdites autre(s) zéolithe(s) est(sont) choisie(s) parmi les zéolithes de structure FAU, seules ou en mélange, de préférence parmi les zéolithes X, et la fraction massique de zéolithe EMT est comprise entre 1 et 50%. Dans un autre mode de réalisation, la fraction massique de zéolithe EMT peut être supérieure à 50% par rapport au poids total d'adsorbant, et de préférence supérieure à 80%. L'adsorbant selon l'invention peut également comprendre des phases zéolithiques d'intercroissance EMT-FAU.

Plus particulièrement, l'adsorbant selon l'invention peut avoir les caractéristiques suivantes :
- la teneur en baryum (exprimée en % poids d'oxyde de baryum BaO) est supérieure à 10%, de préférence supérieure à 15%, de manière très préférée supérieure à 18%, de manière encore plus préférée supérieure à 23%, voire supérieure à 33% en poids par rapport à la masse totale de l'adsorbant. Avantageusement, la teneur en baryum est comprise entre 23% et 40%, bornes incluses, et typiquement entre 33% et 40%,
- la teneur en potassium (exprimée en % poids d'oxyde de potassium K₂O) est comprise entre 0 et 25%, de préférence comprise entre 0 et 15%, de manière encore plus préférée comprise entre 0% et 5% et de manière très préférée de 0% à 2% en poids par rapport à la masse totale de l'adsorbant.

La perte au feu de l'adsorbant selon l'invention, mesurée à 950 °C selon la norme NF EN 196-2 est avantageusement comprise entre 0 et 7,7% et de préférence entre 4,5 et 6,5 % et de manière très préférée entre 4,8 et 6% poids.

Le diamètre moyen en nombre de l'adsorbant selon l'invention peut être compris entre 0,2 mm et 2 mm, en particulier entre 0,4 mm et 0,8 mm et de préférence entre 0,4 mm et 0,65 mm, bornes incluses.

L'invention concerne également un procédé de préparation d'un adsorbant tel que décrit ci-dessus, comprenant au moins les étapes de :
a) agglomération d'une poudre de zéolithe comprenant de la zéolithe EMT, avec un liant et mise en forme, puis séchage et calcination,
b) échange cationique de l'aggloméré par mise en contact avec une solution d'ions baryum, ou d'ions potassium, ou d'ions baryum et d'ions potassium,
c) échange cationique éventuel au potassium,
d) puis lavage et séchage du produit ainsi traité, et
e) activation de l'adsorbant zéolithique ainsi obtenu.

Le procédé de préparation peut comprendre au moins les étapes de :
a) agglomération d'une poudre de zéolithe comprenant de la zéolithe EMT, avec un liant contenant au moins 80 % de préférence, au moins 90%, de préférence encore au moins 95% en poids d'argile zéolithisable et une source de silice, et mise en forme, puis séchage et calcination,
b) zéolithisation dudit liant zéolithisable par action d'une solution basique alcaline, de préférence avec une solution de concentration comprise entre 0,5 M et 5 M et pendant une durée comprise entre quelques dizaines de minutes et quelques heures, de préférence entre environ 1 heure et 8 heures.
c) échange cationique de l'aggloméré par mise en contact avec une solution d'ions baryum, ou d'ions potassium, ou d'ions baryum et d'ions potassium,
d) échange cationique éventuel au potassium,
e) puis lavage et séchage du produit ainsi traité, et
f) activation de l'adsorbant zéolithique ainsi obtenu.

L'invention concerne également l'utilisation dudit adsorbant selon l'invention dans les procédés de :
- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- séparation des alcools polyhydriques,
et notamment pour la séparation de para-xylène à partir de coupes d'isomères aromatiques à 8 atomes de carbone.

L'invention concerne également un procédé de récupération de para-xylène à partir de coupes d'isomères d'hydrocarbures aromatiques contenant 8 atomes de carbone, en phase liquide, par adsorption du para-xylène au moyen dudit adsorbant selon l'invention en présence d'un désorbant, de préférence choisi parmi le toluène et le para-diéthylbenzène.

Ledit procédé peut être de type lit mobile simulé, de préférence à contre-courant simulé.

L'invention concerne également un procédé de récupération de paraxylène à partir de coupes d'isomères d'hydrocarbures aromatiques contenant 8 atomes de carbone, en phase gazeuse, par adsorption du paraxylène au moyen dudit adsorbant selon l'invention en présence d'un désorbant, de préférence choisi parmi le toluène et le para-diéthylbenzène.

### DESCRIPTION DETAILLEE DE L'INVENTION

La présente invention a ainsi pour premier objet des adsorbants zéolithiques à base de zéolithe EMT. Ces adsorbants sont particulièrement adaptés pour une utilisation dans un procédé de séparation du paraxylène en phase liquide, de préférence de type contre-courant simulé.

Ainsi, la présente invention concerne un adsorbant zéolithique comprenant des cristaux de zéolithe EMT, adsorbant qui comprend du baryum et/ou du potassium dans lequel la teneur totale en oxydes d'ions alcalins ou alcalino-terreux autres que le BaO et K₂O est inférieure à 5%, et de préférence entre 0% et 2% et avantageusement entre 0% et 1% en poids, bornes incluses, par rapport à la masse totale de l'adsorbant.

L'adsorbant comprend avantageusement :
- du baryum
- ou du baryum et du potassium.

De manière préférée, l'adsorbant zéolithique est un adsorbant dans lequel :
a. la teneur en oxyde de baryum (BaO) est supérieure à 10 %, de préférence supérieure à 15 %, de manière très préférée supérieure à 18%, de manière plus préférée supérieure à 23 % et de manière encore plus préférée supérieure à 33% en poids par rapport à la masse totale de l'adsorbant,
b. la teneur en oxyde de potassium K₂O est comprise entre 0 et 25%, de préférence comprise entre 0 et 15%, de manière plus préférée entre 0% à 5% et de manière encore plus préférée entre 0% et 2% en poids par rapport à la masse totale de l'adsorbant.

La zéolithe EMT de l'adsorbant de la présente invention peut être préparée de diverses manières. Des procédures de préparation appropriées sont notamment décrites dans les articles suivants : Chem. Mater., (2012), 24, 4758-4765 ; et Science, Jan. 2012, 335, 70-73. Cependant, toute méthode permettant d'obtenir de la zéolithe EMT, avec ou sans structurant organique et avec ou sans ensemencement est également utilisable.

De manière préférée, la zéolithe EMT présente un ratio atomique Si/AI compris entre 1,00 et 2,00 bornes incluses, de préférence entre 1,00 et 1,50 bornes incluses, de manière plus préférée entre 1,05 et 1,50 bornes incluses, et de manière encore plus préférée entre 1,10 et 1,50 bornes incluses. L'invention n'exclut cependant pas que l'adsorbant contienne des mélanges de deux ou plusieurs types de zéolithes EMT tels qu'ils viennent d'être définis.

Dans le présent document, on emploie l'appellation « diamètre moyen en nombre » ou bien « taille » pour les cristaux de zéolithe et pour les agglomérés zéolithiques. La méthode de mesure de ces grandeurs est explicitée plus loin dans la description. Le diamètre moyen en nombre des cristaux de zéolithe EMT est avantageusement compris entre 5 nm et 1500 nm, de préférence compris entre 5 nm et 1000 nm, de manière encore plus préférée entre 10 nm et 500 nm, bornes incluses.

Les adsorbants zéolithiques selon la présente invention comprennent ainsi des cristaux de zéolithe(s) EMT. Les adsorbants zéolithiques selon la présente invention peuvent également contenir des cristaux d'autres zéolithes, choisies parmi les zéolithes de structure FAU, notamment LSX, MSX, X ou Y, EMC-1, ou choisie parmi les zéolithes de structure LTA ou MFI.

Les adsorbants zéolithiques selon la présente invention peuvent également comprendre des phases zéolithiques d'inter-croissance EMT-FAU, telles que les phases CSZ-1, ECR-30, ZSM-20 et ZSM-3, pour ne citer que les principales.

Les adsorbants peuvent également comprendre une phase non zéolithique, c'est-à-dire une phase non cristalline qui est essentiellement inerte vis-à-vis de l'adsorption.

La fraction massique de zéolithe(s) EMT dans l'adsorbant selon la présente invention peut être d'au moins 1% poids de zéolithe(s) EMT par rapport au poids total de l'adsorbant, de préférence d'au moins 10%, de manière plus préférée d'au moins 20%, et de manière encore plus préférée d'au moins 30%. Dans ce cas, l'adsorbant comprend également préférentiellement une autre zéolithe de structure faujasite.

La fraction massique de zéolithe (s) EMT dans l'adsorbant selon la présente invention peut être d'au moins 50% poids de zéolithe(s) EMT par rapport au poids total de l'adsorbant, de préférence d'au moins 80%, cette fraction massique pouvant aller jusqu'à 100% et typiquement jusqu'à 99,5% poids.

L'adsorbant zéolithique de l'invention est de préférence sous la forme d'un aggloméré, c'est-à-dire qu'il est constitué de cristaux de zéolithe(s) et d'au moins une phase non zéolithique qui est un liant d'agglomération permettant la cohésion des cristaux entre eux. Ainsi l'adsorbant zéolithique de l'invention est dénommé « aggloméré » dans la suite du présent exposé.

Selon encore un autre mode de réalisation préférée de l'invention, l'adsorbant zéolithique présente une teneur en oxyde de baryum (BaO) comprise entre 23% et 40%, bornes incluses, et typiquement entre 33% et 40%, bornes incluses, en poids par rapport au poids total de l'adsorbant.

Selon un mode de réalisation préféré, l'adsorbant zéolithique selon l'invention présente une perte au feu mesurée à 950 °C selon la norme NF EN 196-2 comprise entre 0% et 7,7%, préférence entre 4,5 et 6,5 % et avantageusement entre 4,8 et 6%, bornes incluses.

L'adsorbant zéolithique selon la présente invention présente préférentiellement une résistance mécanique généralement supérieure ou égale à 1,8 MPa, typiquement supérieure ou égale à 2,1 MPa. Cette résistance mécanique est mesurée par la méthode Shell série SMS1471-74 adaptée pour des agglomérés de taille inférieure à 1,6 mm.

La capacité d'adsorption est quant à elle mesurée par mesure du volume microporeux de l'adsorbant évalué d'après la méthode du t-plot à partir de l'isotherme d'adsorption d'azote (N₂) à une température de 77K, après dégazage sous vide à 300°C pendant 16 heures. Le volume microporeux des adsorbants zéolithiques de l'invention va préférentiellement de 0,100 cm³/g à 0,350 cm³/g, de préférence de 0,120 cm³/g à 0,350 cm³/g, de préférence encore de 0,140 cm³/g à 0,350 cm³/g.

Selon un autre aspect, l'invention concerne un procédé de préparation des agglomérés zéolithiques tels qu'ils viennent d'être définis, procédé qui comprend au moins les étapes de :
a) agglomération de cristaux d'au moins une zéolithe EMT et éventuellement d'au moins une autre zéolithe avec un liant,
b) optionnellement zéolithisation dudit liant par action d'une solution basique alcaline,
c) échange cationique de l'aggloméré par mise en contact avec une solution d'ions baryum, ou d'ions potassium, ou d'ions baryum et d'ions potassium,
d) échange cationique éventuel au potassium,
e) puis lavage et séchage du produit ainsi traité, et
f) activation de l'aggloméré zéolithique.

La taille des cristaux de zéolithe EMT utilisés à l'étape a) est mesurée par observation au microscope électronique à balayage (MEB) ou par observation au microscope électronique en transmission (MET). Cette observation MEB ou MET permet également de confirmer la présence de phase non zéolithique comprenant par exemple le liant ou le liant résiduel non converti lors de l'étape optionnelle de zéolithisation ou toute autre phase amorphe dans les agglomérés.

L'agglomération et la mise en forme (étape a) peuvent être réalisées selon toutes les techniques connues de l'homme de l'art, telles qu'extrusion, compactage, agglomération, et autres. Les proportions de liant d'agglomération, éventuellement zéolithisable, (voir définition plus loin) et de zéolithe(s) mises en oeuvre sont typiquement celles de l'art antérieur, c'est-à-dire de 5 parties à 20 parties en poids de liant pour 95 parties à 80 parties en poids de zéolithe. Les agglomérés issus de l'étape a), qu'ils soient sous forme de billes, d'extrudés ou autres, ont en général un diamètre moyen en nombre (ou leur plus grande dimension lorsqu'ils ne sont pas sphériques) compris entre 0,2 mm et 2 mm, et en particulier comprise entre 0,4 mm et 0,8 mm et de préférence entre 0,4 mm et 0,65 mm, bornes incluses.

À l'issue de l'étape a), les particules d'agglomérés les plus fines peuvent être éliminées par cyclonage et/ou tamisage et/ou les particules trop grosses par tamisage ou concassage, dans le cas d'extrudés, par exemple.

Le liant d'agglomération mis en œuvre à l'étape a) peut être zéolithisable. Il contient alors au moins 80 % de préférence, au moins 90%, de préférence encore au moins 95%, plus particulièrement au moins 96%, en poids, d'argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique (c'est-à-dire matière active au sens de l'adsorption), le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

D'autres argiles telles que notamment la sépiolite ou l'attapulgite peuvent également être utilisées.

Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

La poudre de zéolithe EMT mise en oeuvre à l'étape a) peut être issue de la synthèse de cristaux de zéolithe(s) EMT comprenant majoritairement, voir exclusivement des cations sodium, par exemple les zéolithes NaEMT, mais on ne sortirait pas du cadre de l'invention en utilisant une poudre ayant subi un ou plusieurs échanges cationiques, après sa synthèse et avant sa mise en oeuvre à l'étape a).

Lors de l'étape a), outre la poudre de zéolithe EMT et le liant, un ou plusieurs additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier. De la silice peut également être ajoutée. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Après le séchage à l'étape a), la calcination est menée à une température en général comprise entre 500°C et 600°C. Dans le cas où la mise en forme est réalisée avec une argile zéolithisable, cette étape permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D.W. Breck, John Wiley and Sons, New York, (1973), p. 314-315.

La zéolithisation du liant d'agglomération est pratiquée selon toute méthode connue de l'homme du métier et peut par exemple être réalisée par immersion du produit de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

L'étape c) d'échange au baryum et/ou au potassium des cations de la zéolithe EMT s'effectue selon les méthodes classiques connues de l'homme du métier, et le plus souvent par mise en contact des agglomérés issus de l'étape b) (ou de l'étape d) avec un sel, tel que le chlorure de baryum (BaCl₂) pour l'échange au baryum et/ou le chlorure de potassium (KCl) pour l'échange au potassium, en solution aqueuse à une température comprise entre la température ambiante et 100°C, et de préférence comprise entre 80°C et 100°C. Pour obtenir rapidement des teneurs en oxyde de baryum élevées, i.e. de préférence supérieures à 10%, plus préférentiellement supérieures à 18% poids par rapport au poids total de l'aggloméré, de manière préférée supérieures 23% en poids et de manière plus préférée allant de 30% à 40% et avantageusement allant de 33% à 40% en poids par rapport au poids total de l'aggloméré, on préfère opérer avec un large excès d'ions baryum par rapport aux cations de la zéolithe que l'on souhaite échanger, typiquement un excès de l'ordre de 10 à 12, avantageusement en procédant par échanges successifs.

L'échange éventuel au potassium (étape d) peut être pratiqué avant et/ou après l'échange au baryum (étape c). Comme indiqué précédemment, il est également possible d'agglomérer à l'étape a) de la poudre de zéolithe EMT contenant déjà des ions potassium (pré-échange des cations présents dans la zéolithe EMT de départ, typiquement cations sodium, par des ions potassium avant l'étape a) et s'affranchir (ou non) de l'étape d).

On procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C. Cette étape e) d'activation a pour but de fixer la teneur en eau, ainsi que la perte au feu de l'adsorbant de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

La présente invention concerne également les utilisations des adsorbants zéolithiques décrits ci-dessus comme agents d'adsorption susceptibles de remplacer avantageusement les agents d'adsorption décrits dans la littérature, à base de zéolithe X, comprenant de l'oxyde de baryum, ou à base de zéolithe X comprenant de l'oxyde de baryum et de l'oxyde de potassium, et notamment dans les utilisations listées ci-dessous :
- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- la séparation des alcools polyhydriques, tels que les sucres.

L'invention concerne notamment un procédé de récupération de para-xylène à haute pureté à partir de coupes d'isomères aromatiques à 8 atomes de carbone consistant à utiliser, comme agent d'adsorption du para-xylène, un adsorbant zéolithique selon l'invention, mis en oeuvre dans des procédés en phase liquide mais aussi en phase gazeuse. Par para-xylène de haute pureté, nous entendons un produit approprié pour une utilisation dans la production d'acide téréphtalique ou de téréphtalate de diméthyle, c'est à dire une pureté d'au moins 99,5% poids, de préférence au moins 99,7% poids, de préférence au moins 99,8% poids et plus préférablement encore au moins 99,9% poids. La pureté du para-xylène peut-être déterminée par des méthodes chromatographiques. Une méthode de chromatographie en phase gazeuse utilisable à la fois pour la détermination de la pureté du para-xylène et des quantités spécifiques d'impuretés est la méthode ASTM D-3798.

On peut ainsi séparer le produit désiré (para-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :
- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (stand alone) et entre 0,7 et 1,4 pour une unité d'adsorption combinée à une unité de cristallisation,
- taux de recyclage (i.e. ratio du débit de recyclage moyen (moyenne des débits de zones pondérée du nombre de lits par zones) sur le débit de charge) compris entre 2,5 et 12, de préférence entre 3,5 et 6.

On pourra sur ce sujet se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le para-diéthylbenzène (PDEB). La sélectivité des adsorbants selon l'invention pour l'adsorption du para-xylène contenu dans des coupes aromatiques en C8 est optimale lorsque leur perte au feu mesurée à 900°C est comprise en général entre 4,0% et 7,7%, et de préférence entre 4,7% et 6,7%.

### TECHNIQUES DE CARACTERISATION

### Granulométrie des cristaux :

L'estimation du diamètre moyen en nombre des cristaux de zéolithe EMT utilisées à l'étape a) et des cristaux de zéolithe EMT contenue dans les agglomérés est réalisée par observation au microscope électronique à balayage (MEB) ou par observation au microscope électronique en transmission (MET).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié, par exemple le logiciel Smile View de l'éditeur LoGraMi. La précision est de l'ordre de 3%.

### Analyse chimique des adsorbants zéolithiques - rapport Si/Al et taux d'échange :

Une analyse chimique élémentaire du produit final obtenu à l'issue des étapes a) à f) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/AI de la zéolithe au sein de l'aggloméré, et de vérifier la qualité de l'échange ionique décrit à l'étape c) et à l'étape optionnelle d). Dans la description de la présente invention, l'incertitude de mesure du ratio atomique Si/AI est de ± 5%.

La qualité de l'échange ionique est liée au nombre de mole d'oxyde de sodium, Na₂O, restant dans l'aggloméré zéolithique après échange. Plus précisément, le taux d'échange par les ions baryum est estimé en évaluant le rapport entre le nombre de moles d'oxyde de baryum, BaO, et le nombre de moles de l'ensemble (BaO + Na₂O). De même, le taux d'échange par les ions baryum et potassium est estimé en évaluant le rapport entre le nombre de moles de l'ensemble oxyde de baryum + oxyde de potassium (BaO + K₂O) et le nombre de moles de l'ensemble (BaO + K₂O + Na₂O). Il est à noter que les teneurs en différents oxydes sont donnés, en pourcentage en poids par rapport au poids total de l'adsorbant zéolithique anhydre.

### Granulométrie des adsorbants zéolithiques :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

### Résistance mécanique des adsorbants zéolithiques :

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 µm qui va permettre notamment de séparer les fines créées lors de l'écrasement. L'utilisation d'un tamis de 425 µm reste adaptée pour des particules de diamètre supérieur à 1,6 mm, mais doit être adaptée selon la granulométrie des agglomérés que l'on cherche à caractériser.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, mieux entre 0,4 mm et 2 mm, et en particulier comprise entre 0,4 mm et 0,8 mm et de préférence entre 0,4 mm et 0,65 mm. Par conséquent, un tamis de 200 µm est utilisé à la place du tamis de 425 µm mentionné dans la méthode Shell standard SMS1471-74.

Le protocole de mesure est le suivant : un échantillon de 20 cm³ d'adsorbants agglomérés, préalablement tamisé avec le tamis adapté (200 µm) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm³ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 200 µm) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

### Détermination des fractions zéolithiques des adsorbants zéolithiques :

La nature et la quantité des différentes fractions zéolithiques sont déterminées par analyse par diffraction de rayons X, connue de l'homme du métier sous l'acronyme DRX. Cette analyse est réalisée sur un appareil de la marque Bruker, puis la quantité des fractions zéolithiques est évaluée au moyen du logiciel TOPAS de la société Bruker. **Volume microporeux** :

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction. Préalablement à l'adsorption, l'adsorbant zéolithique est dégazé entre 300°C - 450°C pendant une durée de 9 heures à 16 heures, sous vide (P < 6,7.10⁻⁴ Pa). La mesure de l'isotherme d'adsorption d'azote à 77K est ensuite effectuée sur un appareil de type ASAP 2010 M de Micromeritics, en prenant au moins 35 points de mesure à des pressions relatives de rapport P/P₀ compris entre 0,002 et 1. Le volume microporeux est déterminé selon la méthode du t-plot à partir de l'isotherme obtenue, en appliquant la norme ISO 15901-3:2007. Le t-plot est le diagramme représentant le volume adsorbé en fonction de l'épaisseur t de multicouche calculée par l'équation de Harkins-Jura. Le volume microporeux est obtenu à partir de l'intersection avec l'axe des ordonnées de la régression linéaire effectuée sur le t-plot pour des valeurs d'épaisseur t comprise entre 0,35 et 0,5nm. Le volume microporeux évalué par la méthode du t-plot est exprimé en cm³ d'adsorbat liquide par gramme d'adsorbant. L'incertitude de mesure est de ± 0,003.

### Perte au feu des adsorbants zéolithiques :

La perte au feu est déterminée en atmosphère oxydante, par calcination de l'échantillon à l'air à une température de 950°C ± 25°C, comme décrit dans la norme NF EN 196-2 (avril 2006). L'écart type de mesure est inferieur à 0,1%.

### Caractérisation de l'adsorption en phase liquide par perçage :

La technique utilisée pour caractériser l'adsorption de molécules en phase liquide sur un solide poreux est la technique dite de perçage, décrite par Ruthven dans « Principles of Adsorption and Adsorption Processes » (Chapitres 8 et 9, John Wiley & Sons, 1984) qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables (équation 9.9). L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

### EXEMPLES

### Exemple A : Synthèse de cristaux de zéolithe EMT

On prépare un gel de composition molaire 18,45 Na₂O-5,15 SiO₂-Al₂O₃-240 H₂O par mélange des réactifs suivants : silicate de sodium, aluminate de sodium, hydroxyde de sodium et eau déminéralisée. Le gel est mûri à 0°C pendant 5 minutes sous agitation. On opère une cristallisation dans un réacteur fermé sous agitation pendant 34 heures à 30°C.

La récupération est faite par centrifugation. Le produit de réaction est d'abord centrifugé 1 fois pour éliminer les eaux-mères (10.000 tours par minute pendant 10 minutes). Le solide obtenu est lavé par repulpage avec de l'eau déminéralisée, puis centrifugé dans les mêmes conditions. Cette opération est renouvelée jusqu'à ce que le pH des eaux de lavage soit inférieur à 8. Le solide est ensuite séché pendant une nuit à 100°C sous air. Les cristaux obtenus sont identifiés par diffraction des rayons X (analyse DRX) comme étant des cristaux de zéolithe EMT. L'analyse chimique du solide donne un rapport atomique Si/AI = 1,2. Le volume microporeux évalué d'après la méthode du t-plot tel que décrit dans la partie technique de caractérisation et exprimé en cm³ par gramme d'adsorbant sec est de 0,14 ± 0,003 cm³/g. L'analyse de la taille des cristaux de zéolithe est réalisée par microscopie électronique en transmission et montre que leur diamètre moyen en nombre est d'environ 20 nm.

### Préparation des adsorbants zéolithiques

### Exemple 1 : Mise en forme par pastillage/concassage

Des agglomérés ont été préparés à partir des cristaux synthétisés (poudre) dans l'exemple A. La poudre est tout d'abord mise en forme par compactage en pastilles. Les pastilles sont ensuite broyées et les agglomérats concassés obtenus sont tamisés afin de conserver ceux dont la granulométrie est comprise entre 0,25 mm et 0,5 mm.

### Exemple 2 : Mise en forme par extrusion/concassage

On prépare un mélange homogène et on agglomère 500 g de cristaux de zéolithe NaEMT préparée selon le mode opératoire décrit dans l'exemple A avec 110 g d'attapulgite (exprimés en équivalent calciné) avec la quantité d'eau qui permet l'extrusion du mélange. Les extrudés sont séchés, concassés de manière à récupérer des grains dont le diamètre moyen en nombre est égal à 0,7 mm, puis calcinés à 550°C sous courant d'azote pendant 2 heures.

### Exemple 3 : Mise en forme par extrusion/concassage et zéolithisation du liant

On prépare un mélange homogène et on agglomère 850 g de cristaux de zéolithe NaEMT préparée selon le mode opératoire décrit dans l'exemple A avec 150 g de kaolin et 30 g de silice colloïdale vendue sous la dénomination commerciale Klebosol^{®}30 (contenant 30% en poids de SiO₂ et 0,5% de Na₂O) avec la quantité d'eau qui permet l'extrusion du mélange. Les extrudés sont séchés, concassés de manière à récupérer des grains dont le diamètre moyen en nombre est égal à 0,7 mm, puis calcinés à 550°C sous courant d'azote pendant 2 heures.

300 g d'agglomérés obtenus sont placés dans un réacteur en verre muni d'une double enveloppe régulée à une température comprise entre 80°C et 100°C, puis on ajoute 2,5 L d'une solution aqueuse d'hydroxyde de sodium de concentration 2,5 M et on laisse le milieu réactionnel sous agitation pendant une durée comprise entre 1 heure et 10 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

### Exemple 4 : Échange cationique au baryum

Les cations sodium des agglomérés obtenus dans les exemples 1, 2 ou 3 sont échangés par des ions baryum au moyen d'une solution aqueuse de chlorure de baryum 0,5 M à 95°C en 4 étapes. À chaque étape, le rapport volume de solution sur masse de solide est de 20 mL/g et l'échange est poursuivi pendant 3 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

La perte au feu mesurée, comme décrit précédemment, est de 5,4% ± 0,1% pour chaque échantillon. Le taux d'échange baryum des agglomérés calculés à partir des analyses élémentaires des oxydes de baryum et de sodium par fluorescence X comme décrit dans les techniques de caractérisation est de 99,7 ± 0,2%.

### Exemple 5 : Échange cationique au baryum et au potassium

Les cations sodium agglomérés obtenus dans les exemples 1, 2 ou 3 sont échangés par des cations baryum et potassium au moyen d'une solution aqueuse de chlorure de potassium 1,35 M et de chlorure de baryum 0,35 M à 95°C en 4 étapes. À chaque étape, le rapport volume de solution sur masse de solide est de 20 mL/g et l'échange est poursuivi pendant 3 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

La perte au feu mesurée, comme décrit précédemment, est de 5,6% ± 0,1% pour chaque échantillon. Le taux d'échange en baryum+potassium des agglomérés calculés à partir des analyses élémentaires des oxydes de baryum et de sodium par fluorescence X comme décrit dans les techniques de caractérisation est de 99,7 ± 0,2%. Le ratio atomique K/Ba est de 0,9 ± 0,1.

### Exemple 6 :Test de perçage

Un test de perçage (chromatographie frontale) est ensuite réalisé sur les agglomérés obtenus à l'exemple 4 pour évaluer leur efficacité. La quantité d'adsorbant utilisée pour ce test est d'environ 30 g.

Le mode opératoire pour obtenir les courbes de perçage est le suivant :
- Remplissage de la colonne par le tamis et mise en place dans le banc de test.
- Remplissage par le solvant à température ambiante.
- Montée progressive à la température d'adsorption sous flux de solvant (5 cm³/min).
- Injection de solvant à 5 cm³/min lorsque la température d'adsorption est atteinte.
- Permutation solvant/charge pour injecter la charge (5 cm³/min).
- L'injection de la charge est ensuite maintenue un temps suffisant pour atteindre l'équilibre thermodynamique.
- Collecte et analyse de l'effluent du perçage.

La pression est suffisante pour que la charge reste en phase liquide, soit 1 MPa. La température d'adsorption est de 175°C. La composition de la charge est la suivante :
- Paraxylène : 22% poids
- Métaxylène : 22% poids
- Orthoxylène : 22% poids
- Éthylbenzène: 22% poids
- Iso-octane : 12% poids (celui-ci est utilisé comme traceur pour l'estimation des volumes non-sélectifs et n'intervient pas dans la séparation)

Les sélectivités binaires du paraxylène par rapport aux autres composés sont calculées à partir des quantités adsorbées de chaque composé, ces dernières étant déterminées par bilan matière à partir des premiers moments des courbes de perçage de l'ensemble des constituants présents dans l'effluent. Les résultats sont consignés dans le tableau 1 ci-dessous :

**Tableau 1**

| ***Sélectivité PX*/*MX*** | ***Sélectivité PX*/*OX*** | ***Sélectivité PX*/*EB*** |
|---|---|---|
| **2,0** | **2,0** | **1,8** |

| | | |
|---|---|---|
| PX : paraxylène, MX : métaxylène, OX : orthoxylène, EB : éthylbenzène | | |

Les résultats montrent donc une sélectivité en faveur du paraxylène. Les facteurs de séparation vis-à-vis du métaxylène, de l'orthoxylène ou de l'éthylbenzène sont proches. Par conséquent, cet adsorbant pourra être avantageusement utilisé pour adsorber sélectivement le paraxylène quelle que soit la composition du mélange à séparer, et notamment dans le cas d'un mélange comportant une fraction importante d'éthylbenzène.

## Revendications

1. Adsorbant zéolithique comprenant des cristaux de zéolithe EMT et comprenant du baryum et/ou du potassium, dans lequel la teneur totale en oxydes d'ions alcalins ou alcalino-terreux autres que l'oxyde de baryum BaO et l'oxyde de potassium K₂O est comprise entre 0 et 5%, bornes incluses, par rapport à la masse totale de l'adsorbant.

2. Adsorbant selon la revendication 1 comprenant en outre une phase non zéolithique.

3. Adsorbant selon l'une des revendication 1 ou 2, dans lequel les cristaux de zéolithes EMT ont un rapport atomique Si/AI compris entre 1,00 et 2,00, bornes incluses.

4. Adsorbant selon l'une des revendications précédentes qui comprend également des cristaux d'au moins une autre zéolithe, choisie parmi les zéolithes de structure FAU, notamment LSX, MSX, X ou Y, EMC-1-, ou choisie parmi les zéolithes de structure, LTA ou MFI.

5. Adsorbant selon l'une quelconque des revendications précédentes, dans lequel la fraction massique de zéolithe EMT est supérieure à 50% par rapport au poids total d'adsorbant.

6. Procédé de préparation d'un adsorbant selon l'une quelconque des revendications précédentes, comprenant au moins les étapes de :
a) agglomération d'une poudre de zéolithe comprenant de la zéolithe EMT, avec un liant et mise en forme, puis séchage et calcination,
b) échange cationique de l'aggloméré par mise en contact avec une solution d'ions baryum, ou d'ions potassium, ou d'ions baryum et d'ions potassium,
c) échange cationique éventuel au potassium,
d) puis lavage et séchage du produit ainsi traité, et
e) activation de l'adsorbant zéolithique ainsi obtenu.

7. Procédé selon la revendication 6 **caractérisé en ce que** le liant mis en oeuvre dans l'étape a) contient au moins 80 % en poids d'argile zéolithisable et une source de silice, et **en ce que** le procédé comprend une étape b) de zéolithisation dudit liant zéolithisable par action d'une solution basique alcaline, de préférence avec une solution de concentration comprise entre 0,5 M et 5 M et pendant une durée comprise entre quelques dizaines de minutes et quelques heures.

8. Utilisation d'un adsorbant selon l'une quelconque des revendications 1 à 5, dans les procédés de :
• séparation de coupes d'isomères aromatiques en C8 et notamment des xylènes,
• séparation d'isomères de toluène substitué tels que nitrotoluène, diéthyltoluène, toluènediamine, et autres,
• séparation des crésols,
• séparation des alcools polyhydriques.

9. Utilisation selon la revendication 8, pour la séparation de para-xylène à partir de coupes d'isomères aromatiques à 8 atomes de carbone.

10. Procédé de récupération de para-xylène à partir de coupes d'isomères d'hydrocarbures aromatiques contenant 8 atomes de carbone, en phase liquide, par adsorption du para-xylène au moyen d'un adsorbant selon l'une quelconque des revendications 1 à 5 en présence d'un désorbant, de préférence choisi parmi le toluène et le para-diéthylbenzène.

11. Procédé de récupération de paraxylène selon la revendication10 de type lit mobile simulé, de préférence à contre-courant simulé.

12. Procédé de récupération de paraxylène à partir de coupes d'isomères d'hydrocarbures aromatiques contenant 8 atomes de carbone, en phase gazeuse, par adsorption du paraxylène au moyen d'un adsorbant selon l'une quelconque des revendications 1 à 5 n présence d'un désorbant, de préférence choisi parmi le toluène et le para-diéthylbenzène.

## Patentansprüche

1. Zeolithadsorptionsmittel mit EMT-Zeolithkristallen und mit Barium und/oder Kalium, wobei der Gesamtgehalt an Alkali- oder Erdalkali-Ionenoxiden mit Ausnahme von Bariumoxid BaO und Kaliumoxid K₂O zwischen 0 und 5%, Grenzen eingeschlossen, mit Bezug auf die Gesamtmasse des Adsorptionsmittels liegt.

2. Adsorptionsmittel nach Anspruch 1, umfassend außerdem eine Nicht-Zeolithphase.

3. Adsorptionsmittel nach einem der Ansprüche 1 oder 2, wobei die EMT-Zeolithkristalle ein Atomverhältnis Si/AI aufweisen, das zwischen 1,00 und 2,00 liegt, Grenzen eingeschlossen.

4. Adsorptionsmittel nach einem der vorhergehenden Ansprüche, das auch Kristalle mindestens eines anderen Zeoliths umfasst, ausgewählt aus den Zeolithen der Struktur FAU, insbesondere LSX, MSX, X oder Y, EMC-1-, oder ausgewählt aus den Zeolithen der Sktruktur LTA oder MFI.

5. Adsorptionsmittel nach einem der vorhergehenden Ansprüche, wobei der Massenanteil des EMT-Zeoliths grösser als 50 % mit Bezug auf das Gesamtgewicht des Adsorptionsmittels ist.

6. Verfahren zur Herstellung eines Adsorptionsmittels nach einem der vorhergehenden Ansprüche, umfassend mindestens die folgenden Schritte:
a) Verfestigen eines Zeolithpulvers mit dem EMT-Zeolith mit einem Bindemittel und Formen, dann Trocken und Kalzinieren,
b) Kationentauschen der Verfestigung durch In-Kontakt-Bringen mit einer Lösung von Bariumionen oder Kaliumionen oder Bariumionen und Kaliumionen,
c) eventuelles Kationentauschen durch Kalium,
d) dann Waschen und Trocknen des derart behandelten Produkts, und
e) Aktivieren des so erhaltenen Zeolithadsorptionsmittels.

7. Verfahren nach Anspruch 6, **dadurch gekennzeichnet, dass** das Bindemittel, implementiert in Schritt a), mindestens 80 Gew% zeolithisierbaren Ton und eine Siliziumquelle enthält, und dadurch, dass das Verfahren einen Schritt b) des Zeolithisierens des zeolithisierbaren Bindemittels durch die Wirkung einer alkalischen basischen Lösung umfasst, vorzugsweise mit einer Lösung einer Konzentration, die zwischen 0,5 M und 5 M liegt, und während einer Dauer, die zwischen einigen zehntel Minuten und einigen Stunden liegt.

8. Verwendung eines Adsorptionsmittels nach einem der Ansprüche 1 bis 5 bei den folgenden Verfahren:
- Trennen von Schnitten von aromatischen Isomeren in C8 und insbesondere der Xylene,
- Trennen von substituierten Toluenisomeren wie z. B. Nitrotoluen, Diethyltoluen, Toluendiamin und anderen,
- Trennen der Kresole,
- Trennen der polyhydrischen Alkohole.

9. Verwendung nach Anspruch 8 zur Trennung vom Paraxylen ausgehend von Schnitten von aromatischen Isomeren mit 8 Kohlenstoffatomen.

10. Verfahren zur Widergewinnung von Paraxylen ausgehend von Schnitten von aromatischen Kohlenwasserstoffisomeren mit 8 Kohlenstoffatomen in flüssiger Phase durch Adsorption des Paraxylens mit Hilfe eines Adsorptionsmittels nach einem der Ansprüche 1 bis 5 in Anwesenheit eines Desorptionsmittels, vorzugsweise ausgewählt aus Toluen und Paradiethylbenzen.

11. Verfahren zum Wiedergewinnen von Paraxylen nach Anspruch 10 vom Typ simuliertes Wanderbett, vorzugsweise mit simuliertem Gegenstrom.

12. Verfahren zum Wiedergewinnen von Paraxylen ausgehend von Schnitten von aromatischen Kohlenwasserstoffisomeren mit 8 Kohlenstoffatomen in flüssiger Phase durch Adsorption des Paraxylens mit Hilfe eines Adsorptionsmittels nach einem der Ansprüche 1 bis 5 in Anwesenheit eines Desorptionsmittels, vorzugsweise ausgewählt aus Toluen und Paradiethylbenzen.

## Claims

1. Zeolite adsorbent comprising zeolite EMT crystals and comprising barium and/or potassium, in which the total content of alkali metal or alkaline-earth metal ion oxides other than barium oxide BaO and potassium oxide K₂O is between 0 and 5%, limits included, relative to the total mass of the adsorbent.

2. Adsorbent according to Claim 1, also comprising a non-zeolite phase.

3. Adsorbent according to Claim 1 or 2, wherein the zeolite EMT crystals have an Si/AI atomic ratio of between 1.00 and 2.00, limits included.

4. Adsorbent according to one of the preceding claims, which also comprises crystals of at least one other zeolite, chosen from zeolites of FAU structure, especially LSX, MSX, X or Y, EMC-1, or chosen from zeolites of LTA or MFI structure.

5. Adsorbent according to one of the preceding claims, in which the mass fraction of zeolite EMT is greater than 50% relative to the total weight of adsorbent.

6. Process for preparing an adsorbent according to any one of the preceding claims, comprising at least the steps of:
a) agglomeration of a zeolite powder comprising zeolite EMT with a binder and forming, followed by drying and calcination,
b) cationic exchange of the agglomerate by placing in contact with a solution of barium ions, or of potassium ions, or of barium ions and potassium ions,
c) optional cationic exchange with potassium,
d) followed by washing and drying of the product thus treated, and
e) activation of the zeolite adsorbent thus obtained.

7. Process according to Claim 6, **characterized in that** step a) uses a binder containing at least 80% by weight of zeolitizable clay and a source of silica and the process comprises a step b) of zeolitization of the said zeolitizable binder by the action of an alkaline basic solution, preferably with a solution with a concentration of between 0.5M and 5M and for a time of between a few tens of minutes and a few hours.

8. Use of an adsorbent according to any one of Claims 1 to 5, in processes of:
• separation of aromatic C8 isomer fractions and especially of xylenes,
• separation of substituted toluene isomers, such as nitrotoluene, diethyltoluene, toluenediamine, and the like,
• separation of cresols,
• separation of polyhydric alcohols.

9. Use according to Claim 8, for the separation of *para*-xylene from aromatic isomer fractions containing 8 carbon atoms.

10. Process for recovering *para*-xylene from aromatic hydrocarbon isomer fractions containing 8 carbon atoms, in the liquid phase, by adsorption of *para*-xylene using an adsorbent according to any one of Claims 1 to 5 in the presence of a desorbent, preferably chosen from toluene and *para*-diethylbenzene.

11. Process for recovering *para*-xylene according to Claim 10 of simulated mobile bed type, preferably of simulated counter-current type.

12. Process for recovering *para*-xylene from aromatic hydrocarbon isomer fractions containing 8 carbon atoms, in the gas phase, by adsorption of *para*-xylene using an adsorbent according to any one of Claims 1 to 5 in the presence of a desorbent, preferably chosen from toluene and *para*-diethylbenzene.
